# EUROPÄISCHE PATENTSCHRIFT

(11) **EP 3 580 739 B1**
(45) Veröffentlichungstag und Bekanntmachung des Hinweises auf die Patenterteilung: **23.04.2025**
(21) Anmeldenummer: 18706219.5
(22) Anmeldetag: 09.02.2018
(51) Int. Cl.: G09B 23/34, G16H 10/65, G16H 50/50, G16H 20/40

(54) **SYSTEM UND VERFAHREN ZUR VALIDIERUNG UND ZUM TRAINING OPERATIVER EINGRIFFE IN DER HUMAN- UND VETERINÄRMEDIZIN**
SYSTEM AND METHOD FOR VALIDATING AND TRAINING SURGICAL INTERVENTIONS IN HUMAN AND VETERINARY MEDICINE
SYSTÈME ET PROCÉDÉ DE VALIDATION ET D'APPRENTISSAGE D'INTERVENTIONS CHIRURGICALES EN MÉDECINE HUMAINE ET VÉTÉRINAIRE

(30) Priorität: 10.02.2017 DE 102017202165; 10.02.2017 DE 102017202164
(43) Veröffentlichungstag der Anmeldung: 18.12.2019
(73) Patentinhaber: Phacon GmbH, 04229 Leipzig (DE)
(72) Erfinder: MÖCKEL, Hendrik, 04159 Leipzig (DE); HAASE, Robert, 04349 Leipzig (DE)
(74) Vertreter: Kailuweit & Uhlemann Patentanwälte Partnerschaft mbB
(86) Internationale Anmeldenummer: PCT/EP2018/053274
(87) Internationale Veröffentlichungsnummer: WO 2018/146250

(56) Entgegenhaltungen:
- DE-U1- 202012 011 452
- GB-A- 2 338 582
- US-A1- 2014 120 505
- ANONYMOUS: "PHACON 3D LIFE MODELLING & SIMULATION Product Catalogue 2016", 30 September 2016 (2016-09-30), pages 1 - 80, XP055464062, Retrieved from the Internet <URL:http://topfermed.com/assets/eng-phacon.pdf> [retrieved on 20180329]

## Beschreibung

Die Erfindung betrifft ein System und ein Verfahren zur Validierung und zum Training operativer Eingriffe in der Human- und Veterinärmedizin, welche ein dem menschlichen oder tierischen Körper anatomisch nachgebildetes Trainingsmodell aufweist.

Dabei werden Parameter festgelegt, die über den Lernerfolg, die Qualität und somit eine Erfolgskontrolle des geübten Eingriffes Auskunft geben. Die geübten Eingriffe umfassen chirurgische und minimal-invasive chirurgische Techniken wie bspw. das Setzen von Implantaten, welche unter realistischen Bedingungen am Trainingsmodell trainiert werden.

Das Trainingsmodell umfasst dabei die anatomische Nachbildung eines Körperteils, eine anatomische Nachbildung einer austauschbaren Übungsregion sowie ein optoelektronisches Detektionsmittel, wobei das optoelektronische Detektionsmittel derart in der Aussparung der anatomischen Nachbildung des Körperteils angeordnet ist, dass dieses zur Erfassung der operativen Eingriffe sowie der Überwachung der Positionierung von operativen Instrumenten und/oder von Implantaten an der Rückseite der austauschbaren Übungsregion ausgebildet ist. Weiterhin umfasst das Trainingsmodell ein Speichermedium, welches lösbar mit der austauschbaren Übungsregion verbunden ist.

Der zu übende operative Eingriff wird durch einen Datensatz unterstützt. Ebenfalls wird eine authentische Verletzung von Risikostrukturen (z.B. von Gefäßen oder Nerven) automatisch angezeigt.

Der Gegenstand des vorliegenden Verfahrens ist die Validierung und das Training operativer Eingriffe in einem dem menschlichen oder tierischen Körper anatomisch nachgebildeten Trainingsmodell mit Hilfe des erfindungsgemäßen Systems.

Zur Ausbildung von Chirurgen und zum vorherigen Erproben von komplizierten chirurgischen und minimal-invasiven chirurgischen Eingriffen vor der eigentlichen Operation sind verschiedene Validierungs- und Trainingsverfahren bekannt, bei welchen Trainingsmodelle verwendet werden. Systeme mit externen Trackingkameras sind z.B. aus dem Katalog "PHACON 3D LIFE MODELLING & SIMULATION Product Catalogue 2016", 30. September 2016 (2016-09-30), Seiten 1-26, XP055464062, bekannt.

In der US 2001/0019818 A1 wird ein anatomisches Modell zum Training von Herz-Operationen mit endoskopischen Methoden beschrieben. Das Modell umfasst anatomisch realistisch nachgebildete Knochen und Organe sowie Hautschichten. Die Organe können zur besseren Darstellbarkeit transparent ausgebildet sein. Zudem wird die Möglichkeit beschrieben, im Inneren des Modells eine Beleuchtung vorzusehen. Das Modell erlaubt jedoch keine automatisierte Kontrolle und Rückkopplung an den Anwender in Bezug auf die vorgenommenen Eingriffe.

Die US 2014/0057236 A1 beschreibt ein kombiniertes System aus einem anatomischen Modell zum Training für chirurgische Eingriffe und einer virtuellen Darstellung des Operationsfeldes. Dabei wird über ein zwischen dem Operateur und dem Modell angeordnetes Display eine virtuelle Darstellung des Operationsfeldes erzeugt, während der Operateur gleichzeitig mit dem darunterliegenden Modell agiert. Dadurch wird eine Gesamtperzeption erzeugt, welche bei dem Operateur ein möglichst realistisches Gefühl erzeugen soll. Das System gibt dem Anwender weiterhin ein Feedback über die richtige Positionierung von Bohrlöchern in Knochen. Dadurch soll der Anwender die exakte Positionierung der Bohrlöcher erlernen können. Nachteilig an dem System ist jedoch die eingeschränkte Perzepterstellung durch die begrenzte virtuelle Darstellung.

Die WO 2012/044753 A2 offenbart ein portables Gerät zum Trainieren laparoskopischer Eingriffe. Das System eignet sich zur Übung von endoskopischen Eingriffen. Das Modell kann auch eine Beleuchtung mittels LED unterhalb der Oberfläche zur Illumination von simulierten Körperhöhlungen aufweisen.

In der WO 2013/165529 A2 wird ein System und ein Verfahren zur Analyse von chirurgischen Techniken offenbart, wobei über optoelektronische Detektionsmittel der Verlauf des chirurgischen Eingriffs erfasst und beurteilt wird. Hierzu befinden sich in einigen Schichten des Modells Farbstoffe, die von dem System erfasst werden.

Die WO 2015/138982 A1 beschreibt ein Modell zum Trainieren von laparskopischen Eingriffen. Dabei orientiert sich der Operateur an der Strukturierung und Ausgestaltung der unter der simulierten Hautschicht befindlichen Muskelschichten. Dadurch wird eine falsche Zugangsstelle identifiziert. Zur Kontrolle der korrekten Einstichstelle kann das Modell umgedreht und von der Rückseite inspiziert werden. Dabei ist jedoch ein Abbruch des Eingriffs notwendig. Zudem ermöglicht dieses Modell keine unmittelbare Kontrolle der Operationstechniken.

Schließlich offenbart die DE 11 2006 003 722 B4 ein Simulationssystem für chirurgische Eingriffe in der Human- und Veterinärmedizin.

Es wäre daher in hohem Maße wünschenswert ein System zur Verfügung zu stellen, welches die ablaufenden Vorgänge innerhalb des Trainingsmodells erfasst und dem Anwender in Echtzeit über den Trainingserfolg und die Positionierung der operativen Instrumente und/oder der Implantate informiert.

Die DE 20 2012 011 452 U1 offenbart ein neurochirurgisches Trainingssystem für die Planung und Durchführung einer Kraniotomie bei Hirntumoren. Das Trainingssystem besteht u.a. aus einem als Schädel ausgebildeten Phantom, einer Steuer-, Mess- und Auswerteeinheit sowie einer Einheit zum Erkennen und Verfolgen des Trackings der beim Training verwendeten Instrumente. Dabei enthält die Steuer-, Mess- und Auswerteeinheit eine Speichereinheit zur Hinterlegung individueller Patientendatensätze.

In DE 10242953 A1 wird eine Vorrichtung zur Schulung von simulierten navigationsunterstützten chirurgischen Eingriffen beschrieben. Dabei werden chirurgische Instrumente durch ein Navigationssystem gesteuert. Weiterhin liegt ein durch bildgebende Verfahren wie bspw. Tomographie-Scans gewonnener Patientendatensatz sowie drei Referenzpunkte, welche nicht in einer Ebene liegen und dem Körper des Patienten positionsfest zugeordnet werden, vor. Das Navigationssystem ist mit einem Modell vom Körper des Patienten ausgestattet, welches drei weitere Referenzpunkte aufweist, die vom Navigationssystem erfassbar sind. Dabei wird der Patientendatensatz dem Modell derart zugeordnet, dass die jeweiligen Referenzpunkte zur Deckung kommen.

Das Dokument DE 10143712 A1 offenbart ein Verfahren, Computersystem und Computerprogrammprodukt zur Datenauswertung und verbesserten Patientenversorgung. Dabei soll die Datenauswertung als internetbasiertes, patientenspezifisches Prognosesystem, insbesondere bei Krebskrankheiten, eingesetzt werden. Der Datensatz enthält dabei klinischpathologische Daten und Informationen zur Nachsorge. Weiterhin wird eine Referenz-Datenbank zum Vergleich herangezogen.

In der DE 10 2008 014 709 A1 ist ein bildgestütztes chirurgisches Expertensystem gezeigt, welches sowohl den Zugriff als auch der Speicherung und dem Austausch von medizinischen Informationen zwischen Bildgebungsgeräten während der Durchführung chirurgischer Prozeduren dient. Dabei werden die erfassten Daten mit Referenzdaten, welche sich bspw. auf eine Krankenakte beziehen, verglichen, um die Prozeduren zu verbessern.

In EP 1348393 A1 wird ein Verfahren zur computergestützten medizinischen Navigation beschrieben. Dabei wird durch eine Positionserfassungseinheit die Position des Patienten sowie die Position von Behandlungsgeräten erfasst. Diesen Positionsdaten werden Körperstrukturdaten zugeordnet. Die Körperstrukturdaten wurden auf der Basis eines generischen Modells erhalten, welches durch Verknüpfung mit patientencharakteristischen Erfassungsdaten angepasst worden ist.

Die DE 10 2013 109 057 A1 offenbart ein Verfahren zur Planung und Vorbereitung eines operativen Eingriffes im menschlichen oder tierischen Körper. Dabei soll wenigstens ein Implantat eingesetzt werden. Durch eine Bildassistenzeinrichtung werden dabei jeweils mehrdimensionale Darstellungen des Körpers gezeigt, welche auf Referenzdatenbanken beruhen.

Schließlich offenbart die DE 112006003722 B4 ein Simulationssystem für chirurgische Eingriffe in der Human- und Veterinärmedizin, bei dem in ein anatomisches Modell Strukturen eingebracht sind, die erlaubte Bereiche und Risikobereiche markieren. In Verbindung mit der Detektion der chirurgischen Instrumente lässt sich so die Annäherung, Berührung und Verletzung von verbotenen Zonen erkennen. Dies wiederum wird durch eine elektronische Steuer-, Mess- und Auswerteeinheit kontrolliert, wodurch gleichzeitig die Erfolgschancen eines geübten operativen Eingriffs bewertet werden.

Nachteilig an diesen Dokumenten ist, dass die Patientendaten auf externen, vom eigentlichen Übungsmodell getrennten Datenbanken gespeichert sind, auf welche ein zeitaufwendiger Zugriff nötig ist, bis die gewünschten Daten verwendet werden können. Zudem enthalten diese Datenbanken die gesamten Parameter aller erfassten Patienten, wodurch der Benutzer erst zeitaufwendig und tlw. unübersichtlich auswählen muss, welchen Datensatz er für die jeweilige Übungssituation verwenden will. Weitere Nachteile sind die zeitaufwendige Aktualisierung der kompletten Datenbanken, welche u.a. auch von der vorhandenen Internetverbindung abhängig sind, sowie bei Störungen der eventuelle Verlust aller Datensätze in der Datenbank. Somit ergibt sich eine uneffektive Verwendung der genannten Systeme.

Es wäre daher in hohem Maße wünschenswert, ein Trainingssystem zur Verfügung zu stellen, welches dem Anwender eine benutzerfreundliche Verwendung gestattet indem dieser das gewünschte durchzuführende Training anhand variabel zusammenstellbarer Komponenten bestimmen kann.

Die Aufgabe der Erfindung ist es daher, ein System bereitzustellen, welches die Nachteile des Stands der Technik überwindet.

Es soll ein Trainingssystem zur Verfügung gestellt werden, welches für die Validierung und zum Training verschieden gestalteter operativer Eingriffe in der Human- und Veterinärmedizin geeignet und dafür individuell angepasst und ausgebildet ist. Durch die individuelle Ausgestaltung des Trainingssystems soll ein effektives, spezialisiertes und fokussiertes Training gewährleistet werden.

Erfindungsgemäß wird die Aufgabe durch ein System, eine anatomische Nachbildung einer austauschbaren Übungsregion und ein Verfahren gemäß den unabhängigen Ansprüchen gelöst. Vorteilhafte Ausgestaltungen sind in den abhängigen Ansprüchen angegeben.

Ein erster Aspekt der Erfindung betrifft ein System zur Validierung und zum Training operativer Eingriffe in der Human- und Veterinärmedizin, welches ein dem menschlichen oder tierischen Körper anatomisch nachgebildetes Trainingsmodell aufweist Das System umfasst eine anatomische Nachbildung eines Körperteils des menschlichen oder tierischen Körpers, welche eine Aussparung aufweist sowie eine anatomische Nachbildung einer austauschbaren Übungsregion, welche in die Aussparung der anatomischen Nachbildung des Körperteils einsetzbar ausgebildet ist und eine Vorderseite aufweist, welche von außen zugänglich ist, und eine Rückseite aufweist, welche formschlüssig mit der Aussparung der anatomischen Nachbildung des Körperteils verbunden ist. Weiterhin umfasst das System ein optoelektronisches Detektionsmittel. Das optoelektronische Detektionsmittel ist dabei derart in der Aussparung der anatomischen Nachbildung des Körperteils angeordnet, dass dieses mittels der Steuer-, Mess- und Auswerteeinheit zur Erfassung der operativen Eingriffe sowie der Überwachung der Positionierung von operativen Instrumenten und/oder von Implantaten an der Rückseite der austauschbaren Übungsregion ausgebildet ist.

Das optoelektronische Detektionsmittel dient dabei dazu die ablaufenden Vorgänge innerhalb des Trainingsmodells zu erfassen und dem Anwender in Echtzeit über den Trainingserfolg und die Positionierung der operativen Instrumente und/oder der Implantate zu informieren. Das Operationsfeld wird somit automatisiert kontrolliert.

Weiterhin umfasst das System eine elektronische Steuer-, Mess- und Auswerteeinheit sowie Mittel zur Signalübertragung.

Das erfindungsgemäße System dient der Evaluation neuentwickelter chirurgischer Systeme und operativer Instrumente, und/oder Implantate der Ausbildung von Chirurgen und zur Erprobung von komplizierten operativen Eingriffen vor der eigentlichen Operation zur Erhöhung der Erfolgschancen eines operativen Eingriffes in verschiedenen chirurgischen Disziplinen.

Der Verlauf und das Ergebnis des operativen Eingriffs werden dabei objektiv und in Echtzeit erfasst und dargestellt.

Das Trainingsmodell dient dem Benutzer, im Folgenden auch Operateur, Chirurg oder Arzt genannt, zur Validierung und zum Training operativer Eingriffe in der Human- und Veterinärmedizin.

In einer Ausführungsform ist das System als Simulationssystem ausgebildet. Das System weist ein Trainingsmodell auf. Dabei werden chirurgische und minimal-invasive chirurgische Techniken durch die Nachbildung von realen anatomischen und funktionellen Bedingungen am Patienten während eines chirurgischen bzw. minimal-invasiven chirurgischen Eingriffes simuliert und getestet.

Das Trainingsmodell stellt eine präzise und anatomisch getreue Nachbildung eines menschlichen oder tierischen Körpers dar.

Erfindungsgemäß umfasst das Trainingsmodell die anatomische Nachbildung eines Körperteils, eine anatomische Nachbildung einer austauschbaren Übungsregion sowie ein optoelektronisches Detektionsmittel.

Erfindungsgemäß umfasst das System eine anatomische Nachbildung eines Körperteils des menschlichen oder tierischen Körpers. Der Begriff "anatomische Nachbildung des Körperteils" bezeichnet im Sinne der Erfindung verschiedene Körperteile, welche alle anatomisch nachbildbar sind. Als Körperteil wird ein morphologisch als funktionelle Einheit erkennbares Segment des Körpers verstanden. Dazu zählen bspw. Bein, Arm, Becken, Kopf oder Wirbelsäule.

In einer Ausführungsform weist die anatomische Nachbildung des Körperteils eines Patienten verschiedene Ausgestaltungen auf. Das entsprechend nachgebildete menschliche oder tierische Körperteil ist dabei anatomisch präzise und real nachgebildet. Die Art der Ausgestaltung des Körperteils basiert auf diversen Faktoren, wie beispielsweise dem nachempfundenen Alter des zu untersuchenden Patienten und/oder der entsprechenden Anatomie. In einer Ausführungsform ist jedes Körperteil eines Menschen oder Tieres als anatomische Nachbildung eines Körperteils darstellbar.

Die Größe der anatomischen Nachbildung eines Körperteils eines Menschen bezieht sich im Sinne der Erfindung auf jede Größe eines Körperteils, die ein Mensch, egal ob männlich oder weiblich, im Laufe seines Lebens annehmen kann. In einer Ausführungsform bezieht sich die Größe des Körperteils auf die nachgebildete realistische Anatomie eines durchschnittlichen erwachsenen Mannes oder einer durchschnittlichen erwachsenen Frau. In einer weiteren Ausgestaltung der Erfindung bezieht sich die Größe des Körperteils auf ein Kind, und entspricht dabei der Größe des Körperteils der jeweils nachgebildeten realistischen Anatomie eines Kindes. Die Größe des Körperteils eines Tieres bezeichnet im Sinne der Erfindung jede Körperteilgröße, die ein Tier, egal ob männlich oder weiblich, im Laufe des Lebens aufweisen kann. In einer alternativen Ausgestaltung der Erfindung ist die Größe des Körperteils kleiner oder größer als die nachgebildete realistische Anatomie bei einem Menschen oder Tier.

Weiterhin basiert die Art der Ausgestaltung der anatomischen Nachbildung eines Körperteils auf den Krankheitsverlauf und/oder das Krankheitsstadium des zu untersuchenden Patienten.

In einer bevorzugten Ausführungsform ist die anatomische Nachbildung des Körperteils als anatomische Nachbildung eines Schädels, ganz besonders bevorzugt als anatomische Nachbildung eines menschlichen Schädels ausgebildet.

Erfindungsgemäß weist die anatomische Nachbildung des Körperteils eine Aussparung auf. Unter einer Aussparung wird im Sinne der Erfindung ein ausgesparter Raum bzw. eine Öffnung oder ein Hohlraum in der anatomischen Nachbildung des Körperteils verstanden. Die Aussparung kann verschiedene Dimensionen aufweisen und ist an die Größe des Körperteils und der in die Aussparung einsetzbaren austauschbaren Übungsregion angepasst. Die Aussparung in der anatomischen Nachbildung des Körperteils entspricht einem Raum, in welchen zumindest ein Teil der austauschbaren Übungsregion einsetzbar ist.

In einer Ausführungsform ist die anatomische Nachbildung eines Körperteils als Schädel ausgebildet, wobei sich die Aussparung im Bereich der Felsenbeinregion des Schädels befindet. In einer alternativen Ausführungsform befindet sich die Aussparung im Bereich der Nasenbeinregion des Schädels.

In einer weiteren alternativen Ausführungsform ist die anatomische Nachbildung eines Körperteils als Arm, in einer speziellen Ausführungsform als Unterarm ausgebildet, wobei sich die Aussparung im Bereich der speichenseitigen und ellenseitigen Arterien befindet.

In einer weiteren alternativen Ausführungsform ist die anatomische Nachbildung eines Körperteils als Rückenregion ausgebildet, wobei sich die Aussparung im Bereich der Wirbelsäule befindet. Dabei kann entweder die ganze Wirbelsäule dargestellt sein oder nur ein Abschnitt wie bspw. die Hals-, Brust- oder Lendenwirbelsäule.

Erfindungsgemäß umfasst das System ein optoelektronisches Detektionsmittel. In einer bevorzugten Ausführungsform ist das optoelektronische Detektionsmittel derart in der Aussparung der anatomischen Nachbildung des Körperteils angeordnet, dass dieses mittels der elektronischen Steuer-, Mess- und Auswerteeinheit zur Erfassung der operativen Eingriffe sowie der Überwachung der Positionierung der operativen Instrumente und/oder der Implantate an der Rückseite der austauschbaren Übungsregion ausgebildet ist.

In einer Ausführungsform dient das optoelektronische Detektionsmittel dazu die ablaufenden Vorgänge innerhalb des Trainingsmodells zu erfassen und dem Anwender in Echtzeit über den Trainingserfolg und die Positionierung der operativen Instrumente und/oder der Implantate zu informieren. Das Operationsfeld wird somit automatisiert kontrolliert.

In einer bevorzugten Ausführungsform ist das optoelektronische Detektionsmittel durch Mittel zur Signalübertragung mit der elektronischen Steuer-, Mess- und Auswerteeinheit verbunden.

In einer Ausführungsform wird ein rückgekoppeltes Signal vom optoelektronischen Detektionsmittel durch Mittel zur Signalübertragung an die elektronische Steuer-, Mess- und Auswerteeinheit gesendet. In einer Ausführungsform handelt es sich bei den Mitteln zur Signalübertragung um elektrische Kabel.

In einer bevorzugten Ausführungsform handelt es sich bei dem optoelektronischen Detektionsmittel um ein Aufnahmegerät, ganz bevorzugt um eine Video-Digitalkamera. In einer alternativen Ausführungsform handelt es sich bei dem optoelektronischen Detektionsmittel um eine Webcam. In einer bevorzugten Ausführungsform hat das optoelektronische Detektionsmittel einen CMOS-Sensor. In einer alternativen bevorzugten Ausführungsform hat das optoelektronische Detektionsmittel einen CCD-Sensor, speziell einem zweidimensionalen CCD-Array-Sensor.

In einer bevorzugten Ausführungsform wird das rückgekoppelte Signal des in der austauschbaren Übungsregion des Trainingsmodells angebrachten optoelektronischen Detektionsmittels an die elektronische Steuer-, Mess- und Auswerteeinheit gesendet und von dem Computerprogrammprodukt ausgewertet und analysiert, sodass der Trainingsverlauf in Echtzeit überwacht und beurteilt wird. Auch die Trainingsdauer und die Anzahl der Verletzungen werden vom Computerprogrammprodukt registriert.

In einer Ausführungsform ist das optoelektronische Detektionsmittel durch Mittel zur Signalübertragung mit der elektronischen Steuer-, Mess- und Auswerteeinheit verbunden. In einer Ausführungsform handelt es sich bei den Mitteln zur Signalübertragung um elektrische Kabel.

In einer Ausführungsform sendet das optoelektronische Detektionsmittel durch die Mittel zur Signalübertragung ein mit dem optoelektronischen Detektionsmittel rückgekoppeltes Signal an die elektronische Steuer-, Mess- und Auswerteeinheit. Das übermittelte Signal enthält Daten über den aktuellen Trainingsverlauf. Die Daten umfassen audiovisuelle Spezifikationen wie Bildauflösung und abgeleitetes Seitenverhältnis, Bildwiederholungsrate, und Farbtiefe.

In einer Ausführungsform überträgt das optoelektronische Detektionsmittel die aufgezeichneten Daten im Betrieb, also während des Trainings, an die elektronische Steuer-, Mess- und Auswerteeinheit. In einer weiteren Ausführungsform überträgt das optoelektronische Detektionsmittel die aufgezeichneten Daten vor oder nach dem Training an die elektronische Steuer-, Mess- und Auswerteeinheit.

Vorteilhaft werden die Daten mit der austauschbaren Übungsregion somit bei Verbinden der austauschbaren Übungsregion mit der anatomischen Nachbildung des Körperteils an die elektronische Steuer-, Mess- und Auswerteeinheit übertragen.

Durch die Anordnung des optoelektronischen Detektionsmittels in der Aussparung der anatomischen Nachbildung des Körperteils werden vorteilhaft die während des Trainings stattfindenden operativen Eingriffe und Techniken durch die Mittel zur Signalübertragung an die elektronische Steuer-, Mess- und Auswerteeinheit übermittelt. Dadurch wird eine Überwachung der Positionierung der operativen Instrumente und/oder der Implantate an der Rückseite der austauschbaren Übungsregion gewährleistet.

In einer Ausführungsform sind die zu untersuchenden Risikostrukturen und/oder der die Risikostrukturen umgebende anatomische Raum transparent ausgestaltet und ermöglichen somit vorteilhaft eine gute Darstellbarkeit für das optoelektronische Detektionsmittel. In einer bevorzugten Ausführungsform sind zumindest Bereiche der austauschbaren Übungsregion transparent ausgebildet. Als Risikostruktur werden jene Komponenten in der austauschbaren Übungsregion des Trainingsmodells bezeichnet, welche während des Trainings verletzt werden können, aber im Einsatz am lebenden Patienten nicht verletzt werden sollten.

In einer Ausführungsform werden als transparentes Material bspw. Kunststoffe wie Polyamide oder Silikone verwendet.

Das optoelektronische Detektionsmittel gewährleistet zudem vorteilhaft eine Erfolgskontrolle des Eingriffes am Trainingsmodell, indem bspw. abgeschätzt werden kann, wie weit ein Implantat wie bspw. ein Cochlea-Implantat eingeführt werden kann. Durch das optoelektronische Detektionsmittel werden die verletzbaren Risikostrukturen und somit Verletzungen in der austauschbaren Übungsregion vorteilhaft überwacht. Weiterhin wird der Anwender durch das optoelektronische Detektionsmittel in Echtzeit über den Trainingserfolg informiert. Zum Trainingserfolg zählt bspw. die Positionierung der operativen Instrumente und/oder der Implantate, bspw. wie weit ein Cochlea-Implantat in die austauschbare Übungsregion eingeführt werden konnte.

Der Benutzer wird durch das rückgekoppelte Signal des optoelektronischen Detektionsmittels in Echtzeit über den Trainingsverlauf, welcher überwacht und beurteilt wird, informiert.

Vorteilhaft kann das Training gegebenenfalls frühzeitig bei auftretenden Fehlern unterbrochen und somit Zeit gespart werden bzw. kann das Training später fortgesetzt bzw. neu gestartet werden.

Unter auftretenden Fehlern wird bspw. ein durch falsche Positionierung der operativen Instrumente und/oder der Implantate an der Vorderseite der austauschbaren Übungsregion hervorgerufener falscher Durchtritt oder Positionierung der operativen Instrumente und/oder der Implantate an der Rückseite der austauschbaren Übungsregion verstanden, welche sich örtlich an einer anderen Stelle der Rückseite der austauschbaren Übungsregion befindet als erforderlich und geplant. Die Anzahl der Verletzungen von funktionell wichtigen anatomischen Arealen, welche durch einen falschen Durchtritt oder Positionierung der operativen Instrumente und/oder der Implantate hervorgerufen wurden, werden dabei registriert.

Weiterhin zählen dazu eine Halterung für das anatomisch nachgebildete Körperteil, damit dieses während des Trainings stabil gelagert und gegen Verrutschen ist.

Während des Eingriffes können vorteilhaft Aussagen über mögliche verletzte Risikostrukturen und somit Verletzungen getroffen werden und so der Operationserfolg verfolgt werden. Die Erfolgskontrolle bzw. der Lernerfolg geben dem Benutzer Sicherheit und Routine bei den operativen Eingriffen. Weiterhin wird vorteilhaft der Verlauf der Operation bewertet. Es wird bspw. eine Bewertung getroffen über die Art der Positionierung der operativen Instrumente und/oder der Implantate bzw. wieviel vom zu entfernenden Gewebe der austauschbaren Übungsregion oder der darin enthaltenen transparent ausgebildeten Risikostrukturen sowie dem die Risikostrukturen umgebenden anatomischen Raum abgetragen wurde.

Erfindungsgemäß umfasst das System eine anatomische Nachbildung einer austauschbaren Übungsregion.

Der Begriff "austauschbare Übungsregion" bezeichnet im Sinne der Erfindung eine Körperregion, welche anatomisch topographisch sowie funktionell der entsprechenden definierten anatomischen Nachbildung eines Körperteils zugeordnet wird. Die austauschbare Übungsregion entspricht dem Operationsfeld für den Benutzer des Trainingsmodells, in welchem er operative Eingriffe übt. In einer Ausführungsform ist die Übungsregion austauschbar. Die austauschbare Übungsregion stellt dabei eine anatomische Nachbildung einer Körperregion dar. In einer Ausführungsform ist jede Körperregion eines Menschen oder Tieres als austauschbare Übungsregion darstellbar.

Erfindungsgemäß ist die austauschbare Übungsregion in die Aussparung der anatomischen Nachbildung des Körperteils einsetzbar ausgebildet. In einer Ausführungsform ist die austauschbare Übungsregion so gestaltet, dass sie formschlüssig in die Aussparung der anatomischen Nachbildung des Körperteils passt.

Weiterhin erfindungsgemäß ist eine anatomische Nachbildung einer austauschbaren Übungsregion in eine Haltevorrichtung einsetzbar ausgebildet. Im Sinne der Erfindung weist die eine anatomische Nachbildung einer austauschbaren Übungsregion eine Vorderseite, welche von außen zugänglich ist, und eine Rückseite, welche formschlüssig mit der Haltevorrichtung verbunden ist, auf. In einer Ausführungsform ist die Haltevorrichtung als technische Halterung ausgebildet. Vorteilhaft lässt sich die anatomische Nachbildung der austauschbaren Übungsregion in jede denkbare Haltevorrichtung formschlüssig einbringen.

In einer bevorzugten Ausführungsform umfasst die in die Haltevorrichtung einsetzbar ausgebildete anatomische Nachbildung einer austauschbaren Übungsregion ein optoelektronisches Detektionsmittel. In einer weiteren bevorzugten Ausführungsform sind zumindest Bereiche der in die Haltevorrichtung einsetzbar ausgebildeten anatomischen Nachbildung einer austauschbaren Übungsregion teilweise transparent ausgebildet. In einer weiteren bevorzugten Ausführungsform umfasst die in die Haltevorrichtung einsetzbar ausgebildete anatomische Nachbildung einer austauschbaren Übungsregion weiterhin ein Speichermedium. In einer weiteren bevorzugten Ausführungsform umfasst die in die Haltevorrichtung einsetzbar ausgebildete anatomische Nachbildung einer austauschbaren Übungsregion Mittel zur Signalübertragung.

In einer bevorzugten Ausführungsform ist die austauschbare Übungsregion lösbar mit der anatomischen Nachbildung des Körperteils verbunden.

In einer bevorzugten Ausführungsform ist die austauschbare Übungsregion als Felsenbein, insbesondere als präzise anatomische Nachbildung der Felsenbeinregion, ganz besonders bevorzugt als menschliche Felsenbeinregion, welche in einen Schädel einsetzbar ist, ausgestaltet. In einer weiteren Ausführungsform ist die austauschbare Übungsregion als präzise anatomische Nachbildung der Nasenbeinregion, welche in einen Schädel einsetzbar ist ausgestaltet. In einer alternativen Ausführungsform ist die austauschbare Übungsregion als präzise anatomische Nachbildung eines Armsegmentes, in einer speziellen Ausführungsform eines Unterarmsegmentes, welches in die Aussparung im Bereich der speichenseitigen und ellenseitigen Arterien einsetzbar ist, ausgestaltet. In einer weiteren alternativen Ausführungsform ist die austauschbare Übungsregion als präzise anatomische Nachbildung eines Wirbelsäulensegmentes, welches in die Aussparung der Rückenregion einsetzbar ist ausgestaltet. Dabei kann entweder die komplette Wirbelsäule dargestellt sein oder nur ein Abschnitt wie bspw. die Hals-, Brust- oder Lendenwirbelsäule.

Die Art der Ausgestaltung der austauschbaren Übungsregion basiert auf diversen Faktoren wie bspw. dem nachempfundenen Alter des Patienten und/oder der entsprechenden Anatomie.

Die Größe der austauschbaren Übungsregion eines Menschen bezieht sich im Sinne der Erfindung auf jede Größe der austauschbaren Übungsregion, die ein Mensch, egal ob männlich oder weiblich, im Laufe seines Lebens annehmen kann. In einer Ausführungsform bezieht sich die Größe der austauschbaren Übungsregion auf die nachgebildete realistische Anatomie eines durchschnittlichen erwachsenen Mannes oder einer durchschnittlichen erwachsenen Frau. In einer weiteren Ausgestaltung der Erfindung bezieht sich die Größe der austauschbaren Übungsregion auf die nachgebildete realistische Anatomie eines Kindes, und entspricht dabei der Größe der Anatomie des jeweiligen kindlichen Alters. Die Größe der austauschbaren Übungsregion eines Tieres bezeichnet im Sinne der Erfindung jede Größe der austauschbaren Übungsregion, die ein Tier, egal ob männlich oder weiblich, im Laufe des Lebens aufweisen kann. In einer alternativen Ausgestaltung der Erfindung ist die Größe der austauschbaren Übungsregion kleiner oder größer als die nachgebildete realistische Anatomie bei einem Menschen oder Tier.

Weiterhin basiert die Art der Ausgestaltung der austauschbaren Übungsregion auf dem Krankheitsverlauf und/oder dem Krankheitsstadium des zu untersuchenden Patienten.

Erfindungsgemäß weist die austauschbare Übungsregion eine Vorderseite auf. In einer Ausführungsform stellt die Vorderseite eine Fläche dar, welche nach außen zeigt und freiliegend ist. Im Sinne der Erfindung ist mit "außen" die Umgebung des Systems gemeint, von welcher der Benutzer freien Zugriff auf die Übungsregion hat. Bevorzugt ist die Vorderseite von außen für den Benutzer zugänglich, d.h. die operativen Instrumente und/oder die Implantate werden durch die Vorderseite in die austauschbare Übungsregion eingeführt. Die Vorderseite der austauschbaren Übungsregion stellt somit den Zugangsbereich für den Benutzer während des Trainings dar.

In einer Ausführungsform erfolgt vor dem Einführen von operativen Instrumenten und/oder Implantaten eine Öffnung der austauschbaren Übungsregion durch ein chirurgisches Verfahren mittels operativer Instrumente. Vorteilhaft wird damit auch das Öffnen der austauschbaren Übungsregion vor bspw. dem Einbringen eines Implantates trainiert. In einer Ausführungsform weist die austauschbare Übungsregion ein Hautimitat auf, welches unterschiedliche und naturgetreue Stärken und mechanische Eigenschaften aufweist, sodass ein reales Durchschneiden des Hautimitates mittels operativer Instrumente erfolgt.

In einer alternativen Ausführungsform befindet sich die Vorderseite der austauschbaren Übungsregion nicht im körperlichen Kontakt mit der anatomischen Nachbildung des Körperteils.

Erfindungsgemäß weist die austauschbare Übungsregion weiterhin eine Rückseite auf.

In einer Ausführungsform stellt die Rückseite eine Fläche dar, welche nach innen, d.h. zur anatomischen Nachbildung des Körperteils, weist.

In einer Ausführungsform befindet sich die Rückseite zumindest teilweise in körperlichem Kontakt mit der anatomischen Nachbildung des Körperteils. Unter körperlichem Kontakt wird erfindungsgemäß eine formschlüssige Verbindung verstanden. In einer Ausführungsform befindet sich die Rückseite zumindest teilweise in körperlichem Kontakt mit der Aussparung der anatomischen Nachbildung des Körperteils. In einer bevorzugten Ausführungsform ist die Rückseite zumindest teilweise formschlüssig mit der Aussparung der anatomischen Nachbildung des Körperteils verbunden. In einer Ausführungsform befindet sich die Rückseite in körperlichem Kontakt mit der Aussparung der anatomischen Nachbildung des Körperteils.

In einer Ausführungsform steht die Rückseite der austauschbaren Übungsregion zumindest teilweise in körperlichem Kontakt mit der anatomischen Nachbildung des Körperteils. In einer Ausführungsform ist die Rückseite von außen für den Benutzer nicht zugänglich.

In einer Ausführungsform sind die anatomische Nachbildung des Körperteils und/oder die austauschbare Übungsregion mittels additiver Fertigungsverfahren auf Basis von dreidimensionalen Patientendaten herstellbar. Beispielsweise ist die anatomische Nachbildung des Körperteils und/oder die austauschbare Übungsregion mittels eines Rapid Prototyping Verfahrens herstellbar. Vorteilhaft lässt sich damit auch eine patientenindividuelle Anatomie der anatomischen Nachbildung des Körperteils bzw. der austauschbaren Übungsregion darstellen, wodurch jedes Training individuell ausgestaltet und durchführbar ist.

In einer bevorzugten Ausführungsform der Erfindung umfasst die austauschbare Übungsregion ein Speichermedium. In einer Ausführungsform umfasst die austauschbare Übungsregion an der Rückseite ein Speichermedium.

Das Speichermedium wird auch als Datenspeicher bezeichnet. In einer Ausführungsform dient ein Halbleiterspeicher als Speichermedium. In einer bevorzugten Ausführungsform ist das Speichermedium ein Speicherchip. In einer Ausführungsform ist das Speichermedium als nichtflüchtiger Datenspeicher wie bspw. als Flash-Speicher ausgebildet.

In einer Ausführungsform sind auf dem Speichermedium Datensätze abgespeichert. In einer bevorzugten Ausführungsform sind auf dem Speichermedium patientenindividuelle Daten gespeichert. In einer weiteren Ausführungsform sind auf dem Speichermedium spezifische Parameter zu der Ausgestaltung der austauschbaren Übungsregion gespeichert.

Durch die bereitgestellten patientenindividuellen Daten wird vorteilhaft ein speziell angepasstes Trainingsmodell an entsprechende Anatomien und Krankheitsgeschichten und damit eine effektive und gezielte Benutzung des Trainingsmodells ermöglicht. Die spezifischen Parameter zu der Ausgestaltung der austauschbaren Übungsregion geben darüber hinaus Hinweise zu der zu untersuchenden Körperregion.

In einer Ausführungsform umfassen die auf dem Speichermedium gespeicherten patientenindividuellen Daten Parameter zur Anatomie des Patienten, zum Alter des Patienten, zu Vorbefunden, zur Pathogenese, zum Krankheitsbild, sowie zu bereits vorhandenen Nachweise von bildgebenden Verfahren wie bspw. CT- oder Röntgenbilder, welche als Vorlagen und zur Orientierung dienen und die Trainingssituation möglichst realistisch erscheinen lassen. In einer Ausführungsform entsprechen die patientenindividuellen Daten der Krankenakte des jeweiligen zu untersuchenden Patienten. Vorteilhaft kann sich der Operateur vor dem Übungseingriff ein Bild über das anstehende und auszuführende Training machen. Somit ist der zu trainierende Eingriff realistischer gestaltet.

In einer weiteren Ausführungsform sind auf dem Speichermedium spezifische Parameter zu der Ausgestaltung der austauschbaren Übungsregion und damit der zu untersuchenden Körperregion gespeichert. Vorteilhaft liegt damit die zu untersuchende Körperregion nicht nur als anatomische Nachbildung der austauschbaren Übungsregion vor, sondern umfasst auch alle benötigten spezifischen Parameter (Ausgestaltung und Geometrie der austauschbaren Übungsregion) zur Durchführung des Trainings.

In einer bevorzugten Ausführungsform ist das Speichermedium mit weiteren patientenindividuellen Daten beschreibbar oder überschreibbar. Dies erfolgt, indem diese zusätzlich auf das Speichermedium geschrieben und ergänzt oder die ursprünglich vorhandenen patientenindividuellen Daten durch diese überschrieben werden. Unter weiteren patientenindividuellen Daten werden entweder erweitere Befunde desselben Patienten oder die neuen Daten eines anderen Patienten mit einem anderen Krankheitsbild und Anatomie und Alter verstanden, welche komplett neu oder zusätzlich auf das Speichermedium geschrieben und abgespeichert werden. Somit kann der Benutzer stets neue Eingriffe am Trainingsmodell üben. Weiterhin ist die Steuerelektronik des Speichermediums stets aktualisierbar, wodurch Fehler wie Manipulationen oder Ausfälle reduziert werden.

In einer Ausführungsform ist das Speichermedium mechanisch lösbar mit der austauschbaren Übungsregion verbunden. In einer bevorzugten Ausführungsform ist das Speichermedium lösbar in der austauschbaren Übungsregion angeordnet. In einer weiteren Ausführungsform ist das Speichermedium lösbar in der Rückseite der austauschbaren Übungsregion angeordnet. Vorteilhaft kann das Speichermedium somit von der austauschbaren Übungsregion zum Beschreiben mit weiteren oder neuen patientenindividuellen Daten, zum Beschreiben mit weiteren oder neuen spezifischen Parametern zu der Ausgestaltung der austauschbaren Übungsregion oder zur Aktualisierung der Steuerelektronik entfernt und an diese wieder angeschlossen werden.

Durch die lösbare Anordnung des Speichermediums in der austauschbaren Übungsregion ist es möglich, verschiedene Speichermedien, welche jeweils unterschiedliche patientenindividuelle Daten und spezifische Parameter zu der Ausgestaltung der austauschbaren Übungsregion enthalten, an die austauschbare Übungsregion anzuschließen und somit verschiedene Krankheitsbilder für die gleiche ausgebildete anatomische austauschbare Übungsregion zu trainieren.

In einer Ausführungsform ist das Speichermedium mechanisch und elektrisch lösbar mit der anatomischen Nachbildung des Körperteils verbunden.

In einer bevorzugten Ausführungsform ist das Speichermedium durch Mittel zur Signalübertragung mit der elektronischen Steuer-, Mess- und Auswerteeinheit verbunden.

Erfindungsgemäß ist die austauschbare Übungsregion in die Aussparung der anatomischen Nachbildung des Körperteils einsetzbar ausgebildet. In einer Ausführungsform ist die austauschbare Übungsregion dabei in die Aussparung der anatomischen Nachbildung des Körperteils formschlüssig lösbar ausgebildet. In einer Ausführungsform wird die austauschbare Übungsregion mechanisch mit der anatomischen Nachbildung des Körperteils verbunden.

Von der anatomischen Nachbildung des Körperteils abgehend sind Mittel zur Signalübertragung, welche wiederum mit der elektronischen Steuer-, Mess- und Auswerteeinheit verbunden sind.

In einer Ausführungsform handelt es sich bei den Mitteln zur Signalübertragung um elektrische Kabel. In einer alternativen Ausführungsform handelt es sich bei den Mitteln zur Signalübertragung um eine drahtlose Verbindung, welche beispielsweise als Bluetooth oder WLAN ausgebildet ist.

Die anatomische Nachbildung des Körperteils und das Speichermedium der austauschbaren Übungsregion bilden eine lösbare Steckverbindung. Dadurchwerden eine elektrische Verbindung sowie eine Datenverbindung, speziell als Hardwareschnittstelle, sichergestellt.

In einer bevorzugten Ausführungsform wird die anatomische Nachbildung des Körperteils mit der austauschbaren Übungsregion verbunden und das in der austauschbaren Übungsregion enthaltene Speichermedium mit den darauf bereit gestellten patientenindividuellen Daten und den spezifischen Parametern zu der Ausgestaltung der austauschbaren Übungsregion von der elektronischen Steuer-, Mess- und Auswerteeinheit erkannt. Dies geschieht, indem durch Mittel zur Signalübertragung die patientenindividuellen Daten sowie die spezifischen Parameter zu der Ausgestaltung der austauschbaren Übungsregion an die elektronische Steuer-, Mess- und Auswerteeinheit übertragen und zugeordnet werden. Damit ist ein Datenaustausch beim Verbinden von dem Speichermedium in der austauschbaren Übungsregion mit der anatomischen Nachbildung des Körperteils an die elektronische Steuer-, Mess- und Auswerteeinheit gewährleistet.

Vorteilhaft wird somit durch das Speichermedium ein zeitaufwendiger Zugriff auf eine komplette Datenbank wie bspw. in DE 20 2012 011 452 U1 beschrieben, welche alle patientenindividuellen Daten enthält und diese bspw. erst laden bzw. übermitteln muss, umgangen. Vorteilhaft ist ein schneller und unabhängiger Zugriff auf die patientenindividuellen Daten und spezifischen Parameter der austauschbaren Übungsregion gewährleistet, ohne dass bspw. eine komplette Datenbank aktualisiert werden muss.

Auf die auf dem Speichermedium enthaltenen patientenspezifischen Daten und spezifischen Parameter zu der Ausgestaltung der austauschbaren Übungsregion ist vorteilhaft jederzeit ein schneller Zugriff möglich. Das System ist nicht abhängig von einer zentralen Datenbank, auf welche der Zugriff bspw. durch eine Internetverbindung ist und es somit erforderlich wäre, eine stets verlässliche Verbindung zu gewährleisten. Vorteilhaft kann das Trainingsmodell somit auch an Orten ohne Internetverbindung verwendet werden. Weiterhin sind die patientenindividuellen Daten und die spezifischen Parameter zu der Ausgestaltung der austauschbaren Übungsregon bspw. im Falle eines Systemabsturzes vorteilhaft nicht betroffen, da sie nicht zentral in einer Datenbank, sondern auf den Speichermedien abgespeichert sind.

Durch Übermittlung und Darstellung der patientenindividuellen Daten beim Verbinden der austauschbaren Übungsregion mit der anatomischen Nachbildung des Körperteils wird sofort erkannt, um welchen Patienten (Alter, Anatomie, Vorbefunde, Pathogenese, Krankheitsbild, bereits vorhandene Nachweise von bildgebenden Verfahren wie bspw. CT- oder Röntgenbilder) es sich handelt und welche operativen Eingriffe der Benutzer vorzunehmen hat. Durch gleichzeitige Übermittlung und Darstellung der spezifischen Parameter über die jeweilige Ausgestaltung der zu untersuchenden austauschbaren Übungsregion beim Verbinden der austauschbaren Übungsregion mit der anatomischen Nachbildung des Körperteils wird weiterhin sofort erkannt, um welche anatomische Nachbildung der Körperregion es sich handelt.

In einer bevorzugten Ausführungsform umfasst das System operative Instrumente. In einer bevorzugten Ausführungsform umfassen die bereitgestellten operativen Instrumente verschiedene Werkzeuge aus dem chirurgischen und minimal-chirurgischen Bereich wie bspw. Zeigewerkzeug, Bohrer, Kugelstopfer, chirurgische Schere, chirurgische Zange, Sauger oder medizinisches Endoskop.

Vorteilhaft werden die operativen Instrumente vom Benutzer exakt an der gewünschten und medizinisch erforderlichen Stelle positioniert.

In einer Ausführungsform wird das oder werden die ausgewählten und medizinisch erforderlichen operativen Instrumente auf der Vorderseite der austauschbaren Übungsregion positioniert und durch die austauschbare Übungsregion hindurch zur Rückseite der austauschbaren Übungsregion geführt. Der Durchtritt und die Positionierung der operativen Instrumente an der Rückseite der austauschbaren Übungsregion ist als Einstichstelle im Hautimitat sichtbar.

In einer Ausführungsform enthalten die operativen Instrumente eine Markierung, welche sie kennzeichnende Merkmale und Parameter enthält. Vorteilhaft ist die Markierung unterschiedlich gestaltet, sodass die operativen Instrumente unterscheidbar sind. Die Markierung auf den operativen Instrumenten wird von einem Detektionsmittel wie einer Kamera erfasst und an ein Computerprogrammprodukt weitergeleitet, wodurch erkannt wird, welche operativen Instrumente beim jeweiligen Training verwendet werden. In einer Ausführungsform überwacht das Detektionsmittel zudem den operativen Eingriff auf der Vorderseite der austauschbaren Übungsregion.

In einer Ausführungsform ist jedes handelsüblich ausgebildete operative Instrument für das Training operativer Eingriffe mit dem erfindungsgemäßen System geeignet. In einer Ausführungsform sind die operativen Instrumente als Aufsätze ausgestaltet.

In einer Ausführungsform sind die operativen Instrumente durch Mittel zur Signalübertragung mit der elektronischen Steuer-, Mess- und Auswerteeinheit verbunden. In einer Ausführungsform handelt es sich bei den Mitteln zur Signalübertragung um elektrische Kabel.

Die operativen Instrumente sind individuell an das auszuführende Training angepasst und können je nach Art des Trainings oder im Falle von Reparaturen vorteilhaft ausgetauscht werden.

In einer bevorzugten Ausführungsform umfasst das System Implantate. In einer Ausführungsform umfassen die Implantate medizinische Implantate wie bspw. ein Cochleaimplantat.

Vorteilhaft werden die Implantate vom Benutzer exakt an der gewünschten und medizinisch erforderlichen Stelle positioniert.

In einer Ausführungsform wird das oder werden die ausgewählten und medizinisch erforderlichen Implantate auf der Vorderseite der austauschbaren Übungsregion positioniert und durch die austauschbare Übungsregion hindurch zur Rückseite der austauschbaren Übungsregion geführt. Der Durchtritt und die Positionierung der Implantate an der Rückseite der austauschbaren Übungsregion ist als Einstichstelle im Hautimitat sichtbar.

In einer Ausführungsform sind die Implantate durch Mittel zur Signalübertragung mit der elektronischen Steuer-, Mess- und Auswerteeinheit verbunden. In einer Ausführungsform handelt es sich bei den Mitteln zur Signalübertragung um elektrische Kabel.

Die Implantate sind individuell an das auszuführende Training angepasst und können je nach Art des Trainings oder im Falle von Reparaturen vorteilhaft ausgetauscht werden.

In einer bevorzugten Ausführungsform umfasst das System eine elektronische Steuer-, Mess- und Auswerteeinheit. Durch die elektronische Steuer-, Mess- und Auswerteeinheit wird eine virtuelle und/oder realistische Darstellung der Untersuchung im Operationsfeld beim Training am Trainingsmodell gewährleistet.

In einer bevorzugten Ausführungsform umfasst das System Mittel zur Signalübertragung. Die elektronische Steuer-, Mess- und Auswerteeinheit ist mit Mitteln zur Signalübertragung verbunden. Dadurch wird eine automatisierte Kontrolle der austauschbaren Übungsregion und des Operationsfeldes im Betrieb, also während des Trainings, ermöglicht. In einer Ausführungsform handelt es sich bei den Mitteln zur Signalübertragung um elektrische Kabel.

Unter einer virtuellen Darstellung wird eine dreidimensionale Wiedergabe des Trainingsverlaufs im Operationsfeld der austauschbaren Übungsregion verstanden, welche durch das Computerprogrammprodukt generiert wird. Dabei dient medizinisches Bildmaterial, welches auf dem Speichermedium hinterlegt ist, als Grundlage für die virtuelle Darstellung, welche von einem Computerprogrammprodukt generiert wird.

Unter einer realistischen Darstellung wird eine visuelle Wiedergabe des Trainingsverlaufs im Operationsfeld der austauschbaren Übungsregion verstanden, welche bspw. durch ein optoelektronisches Detektionsmittel als Videobild in Echtzeit übertragen wird.

Die virtuelle und/oder realistische Darstellung des Operationsfeldes wird an die elektronische Steuer-, Mess- und Auswerteeinheit übermittelt, während der Benutzer gleichzeitig mit dem darunterliegenden Trainingsmodell agiert.

In einer Ausführungsform handelt es sich bei der elektronischen Steuer-, Mess- und Auswerteeinheit um einen Computer.

In einer Ausführungsform umfasst die elektronische Steuer-, Mess- und Auswerteeinheit weiterhin ein Computerprogrammprodukt, welches für die virtuelle und/oder realistische Darstellung des Operationsfeldes und Trainingsverlaufs sorgt.

In einer Ausführungsform können die zu lernenden chirurgischen Szenarien anhand von Ablauf-Protokollen studiert werden. In den Ablauf-Protokollen werden Parameter festgelegt, die über die Qualität des geübten Eingriffes und damit (bei mehreren Tests) über den Lernerfolg als Erfolgskontrolle Auskunft geben. Bei den Parametern handelt es sich bspw. um die Durchführungsdauer, economy of hand-movement, Verletzung von funktionell wichtigen anatomischen Arealen.

In einer Ausführungsform werden durch das Computerprogrammprodukt weiterhin die beim Training verwendeten operativen Instrumente und/oder Implantate aufgrund der an ihnen angebrachten Markierung erkannt und entsprechend für die jeweilige Trainingssituation kalibriert. Somit werden das Training und der Trainingsverlauf am Trainingsmodell gesteuert und kontrolliert.

In einer Ausführungsform wird die Kalibrierung der operativen Instrumente vorgenommen, um die Achsenlängen und den Ort der Spitze der operativen Instrumente zu bestimmen. Weiterhin findet eine Richtungskalibrierung statt, bei welcher die Orientierung der operativen Instrumente innerhalb der virtuellen und/oder realistischen Darstellung des Operationsfeldes zu bestimmen.

In einer weiteren Ausführungsform wird durch das Computerprogrammprodukt die entsprechend verwendete austauschbare Übungsregion mit den auf ihrem Speichermedium enthaltenen spezifischen Parametern zur Ausgestaltung der austauschbaren Übungsregion sowie patientenindividuelle Daten bei der Verbindung der austauschbaren Übungsregion mit der anatomischen Nachbildung des Körperteils erkannt.

In einer weiteren Ausführungsform kann eine Patienten-Kalibrierung vorgenommen werden, bei welcher das in der virtuellen und/oder realistischen Darstellung erzeugte Koordinatensystem der in der Aussparung der anatomischen Nachbildung des Körperteils befindlichen austauschbaren Übungsregion an dem Koordinatensystem der operativen Instrumente ausgerichtet wird.

In einer bevorzugten Ausführungsform wird das rückgekoppelte Signal des in der austauschbaren Übungsregion des Trainingsmodells angebrachten optoelektronischen Detektionsmittels an die elektronische Steuer-, Mess- und Auswerteeinheit gesendet und von dem Computerprogrammprodukt ausgewertet und analysiert, sodass der Trainingsverlauf in Echtzeit überwacht und beurteilt wird. Auch die Trainingsdauer und die Anzahl der Verletzungen werden vom Computerprogrammprodukt registriert.

In einer bevorzugten Ausführungsform wird das erfindungsgemäße System und das erfindungsgemäße Verfahren zur Validierung und zum Training operativer Eingriffe in der Human- und Veterinärmedizin verwendet. In einer Ausführungsform ist das System für Auszubildende, Fachärzte, Trainierenden oder Systemtestern zum Üben vorgesehen. In einer weiteren Ausführungsform ist das System für Unternehmen vorgesehen, welche ihre eigenen operativen Instrumente und/oder Implantate einsetzen, verwenden und/oder den Umgang mit diesen demonstrieren.

Die operativen Eingriffe umfassen chirurgische sowie minimal-invasive chirurgische Eingriffe.

Die operativen Eingriffe werden durch die bereitgestellten operativen Instrumente und/oder Implantate realisiert.

Zu den zu trainierenden chirurgischen Eingriffen zählen bspw. das Abtragen von Knochen, das die Insertion von medizinischen Implantaten, das Setzen von medizinischen Schrauben, die Verschaffung von Zugängen, die Entfernung von Gewebe wie bspw. Tumoren sowie das Schaffen operativer Zugänge zu den erkrankten Regionen.

Zu den zu trainierenden chirurgischen Eingriffen zählen auch minimal-invasive Methoden wie bspw. die medizinische Endoskopie, die minimal-invasive Wirbelsäulenchirurgie (u.a. die Dekompression von Rückenmarksbereichen und Fusionierung von Wirbeln nach Bandscheibenvorfällen), das Setzen von Implantaten sowie die Entfernung von Tumoren und/oder Knochenverwachsungen.

Vorteilhaft ist durch das erfindungsgemäße System ein effektives Training für den Benutzer möglich. Durch das optoelektronische Detektionsmittel kann das Training in Echtzeit überwacht werden. Durch die individuelle anatomische Gestaltung sowie die patientenindividuellen Daten auf dem Speichermedium kann der Benutzer sich auf eine individuelle und spezielle Problemstellung fokussieren.

Vorteilhaft umfasst das Trainingsmodell austauschbare Komponenten wie spülbares, knochenähnliches Material sowie pneumatisierte Knochen.

Zu den typischen zu übenden chirurgischen Eingriffe in der Felsenbeinregion zählen das Training der Mastoidektomie und Cochleostomie sowie das Setzen von Implantaten wie bspw. Mittel- und Innenohrimplantaten.

Die künstlichen Nasennebenhöhlenpatienten ermöglichen vor allem das Training der Schädelbasischirurgie sowie der Funktionalen Endoskopischen Sinus-Chirurgie (FESS), bei welcher u.a. die Eröffnung der Nasennebenhöhlen und das Entfernen von Tumoren (Hypophysentumor) oder Polypen zu den häufigen zu trainierenden Eingriffen zählen.

Bei der Wirbelsäulenchirurgie kommen trainingsintensive Eingriffe sehr häufig bei der Operation an Bandscheiben bei einer Dekompression oder bei der Fixierung der Wirbelkörper (bspw. nach einer Fraktur) vor. Weiterhin können mit Hilfe des Trainingsmodells minimalinvasive OP-Techniken und manuelle Fähigkeiten wie das Setzen von Pedikelschrauben und Zwischenwirbelimplantaten (Cages) sowie der Fusionierung von Wirbeln erlernt werden.

Durch die Verwendung des Trainingsmodells werden die Qualität und der Lernerfolg der operativen Eingriffe validiert. Durch diese Erfolgskontrolle wird dem Benutzer sofort mitgeteilt, ob Risikostrukturen verletzt wurden oder die Operation ohne Störungen verlief.

In Ausführungsformen der Erfindung sind die Komponenten des Trainingsmodells wiederverwendbar ausgebildet.

Je nach Art des ausgeführten Trainings sind alle bzw. manche Komponenten des Trainingsmodells wiederverwendbar. Nach dem Training wird die austauschbare Übungsregion aus der anatomischen Nachbildung des Körperteils entnommen und beide Komponenten separat gereinigt. Die anatomische Nachbildung des Körperteils wird aus der Halterung genommen und auch die Halterung für die anatomische Nachbildung des Körperteils wird gereinigt. Weiterhin werden die verwendeten operativen Instrumente gesäubert.

Für die Realisierung der Erfindung ist es auch zweckmäßig, die vorbeschriebenen erfindungsgemäßen Ausgestaltungen, Ausführungsformen und Merkmale der Ansprüche in jeder Anordnung miteinander zu kombinieren.

Nachfolgend soll die Erfindung anhand einiger Ausführungsbeispiele eingehender erläutert werden. Die Ausführungsbeispiele sollen dabei die Erfindung beschreiben ohne diese zu beschränken.

Die Erfindung ist auf nahezu allen Gebieten der Human- und Veterinärmedizin anwendbar. Anhand von Zeichnungen wird die Erfindung näher erläutert. Dabei zeigen
- Fig. 1: eine anatomische Nachbildung eines Schädels mit austauschbarer Felsenbeinregion und optoelektronischem Detektionsmittel,
- Fig. 2: eine Detailansicht der anatomischen Nachbildung eines Schädels mit austauschbarer Felsenbeinregion und optoelektronischem Detektionsmittel,
- Fig. 3: eine Einzelansicht der austauschbaren Felsenbeinregion mit optoelektronischem Detektionsmittel,
- Fig. 4: eine austauschbare Felsenbeinregion mit Speichermedium und Anschlüssen für die Mittel zur Signalübertragung an die elektronische Steuer-, Mess- und Auswerteeinheit,
- Fig. 5: die austauschbare Felsenbeinregion aus Fig. 4 in einer Seitenansicht,
- Fig. 6: eine austauschbare Nasenregion von der Seite in einer Sprengskizze mit Speichermedium und Anschlüssen für die Mittel zur Signalübertragung an die elektronische Steuer-, Mess- und Auswerteeinheit,
- Fig. 7: die austauschbare Nasenregion aus Fig. 6 in einer Frontansicht,
- Fig. 8: eine anatomische Nachbildung eines Schädels mit austauschbarer Nasenregion und Speichermedium sowie Anschlüssen für die Mittel zur Signalübertragung an die elektronische Steuer-, Mess- und Auswerteeinheit.

Das erste Ausführungsbeispiel bezieht sich auf die Felsenbeinregion und somit auf ein begrenztes Gebiet des Wirkungsbereiches von HNO-Ärzten. Das Trainingsmodell wird für eine Implantation einer Elektrode eines Cochlea-Implantats verwendet und umfasst die anatomische Nachbildung eines menschlichen Schädels, in deren Aussparung eine anatomisch geformte und austauschbare Felsenbeinregion einsetzbar ist. Die Felsenbeinregion wird mit dem Schädel passgenau verbunden.

Dabei umfasst die Felsenbeinregion einen Abschnitt des Schläfenbeins (Os temporale) und umgibt das Innenohr (Labyrinth). Weiterhin zählen auch das Pars tympanica (umgibt das Mittelohr) und Pars mastoidea des Schläfenbeins zur Felsenbeinregion. Zu der Felsenbeinregion zählen weiterhin der Cochlea mit Scala tympani und Bogengängen sowie (bewegliche) Gehörknöchelchen. Das Weichgewebe ist aus Silikon geformt wie bspw. die Nachbildung der Dura. Die Felsenbeinregion umfasst weiterhin eine Nachbildung des Trommelfells sowie Muskeln und Nerven wie bspw. den nervus dacialis, Chorda typani und Musculus stapedius. Auch Blutgefäße wie die Vena jugularis und Arteria carotis werden nachgebildet.

Durch die Felsenbeinregion ziehen unter anderem der Nervus facialis und der Nervus tympanicus. An der Vorderfläche (bei Tieren der Innenfläche) der Felsenbeinregion liegt eine seichte Grube für das Ganglion trigeminale. Die Felsenbeinregion besitzt drei wichtige Zugänge: Das Porus acusticus internus (innere Gehöröffnung), an welchem der Nervus facialis eintritt und der Nervus vestibulocochlearis austritt, das Foramen stylomastoideum, an welchem der Nervus facialis austritt sowie das Canalis musculotubarius, dem Kanal der Ohrtrompete (Tuba auditiva) ins Mittelohr. Weiterhin dient eine Spalte (Fissura petrotympanica) zwischen der Felsenbeinregion und dem Paukenteil (Pars tympanica) des Schläfenbeins dem Austritt der Paukensaite (Chorda tympani). Bei einigen Säugetieren (z. B. Mensch, Pferd, Rind) ist das Zungenbein am Griffelfortsatz (Processus styloideus) des Felsenbeins ligamentär befestigt.

Die Felsenbeinregion weist eine Vorderseite, welche von außen für den Operateur frei zugänglich ist, sowie eine Rückseite auf. Dabei steht die Rückseite der Felsenbeinregion zumindest teilweise in körperlichem Kontakt mit der anatomischen Nachbildung des Schädels.

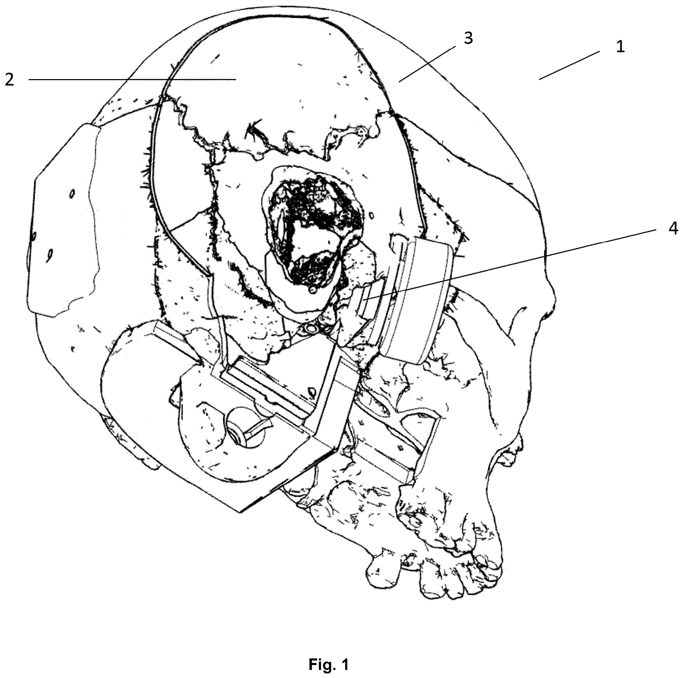

Figur 1 zeigt eine Übersichtsskizze des erfindungsgemäßen Systems 1. Als austauschbare Übungsregion 2 ist die Felsenbeinregion gezeigt. Diese ist als sensibel detektierte Struktur in der anatomischen Nachbildung eines Körperteils 3, hier einem menschlichen Schädel, formschlüssig eingesetzt. Ein optoelektronisches Detektionsmittel 4, hier eine Videodigitalkamera, ist über Mittel zur Signalübertragung 7 (nicht gezeigt) mit der austauschbaren Übungsregion 2 und einer elektronischen Steuer-, Mess- und Auswerteeinheit 5 (nicht gezeigt) verbunden.

In der Aussparung des Schädels ist eine (Video-)Digitalkamera (nicht gezeigt) so angeordnet, dass der operative Eingriff durch einen Computer (nicht gezeigt) erfasst wird und weiterhin die Positionierung der operativen Instrumente und/oder der Implantate (nicht gezeigt) durch den Computer (nicht gezeigt) an der Rückseite der Felsenbeinregion überwacht wird.

Eine SSD-Karte dient als Speichermedium (nicht gezeigt). Die SSD-Karte ist an der Rückseite des austauschbaren Felsenbeins befestigt.

Alle stattfindenden Prozesse werden auf einem Monitor (nicht gezeigt) dargestellt. Die (Video-) Digitalkamera sendet dabei ein rückgekoppeltes Signal an den Computer (nicht gezeigt).

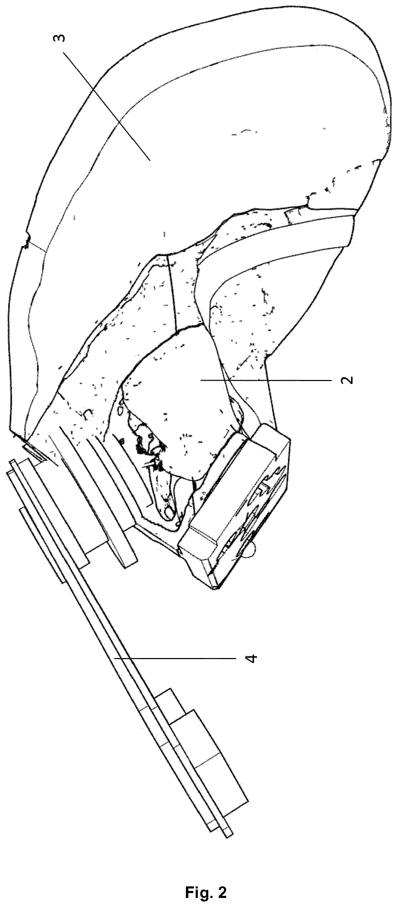

Figur 2 zeigt eine Detailansicht des Systems 1 der anatomischen Nachbildung eines Schädels 3 mit einer darin formschlüssig eingesetzten austauschbaren Region des Felsenbeines des Innenohres 2 als sensibel detektierte ("gläserne") Struktur sowie ein optoelektronisches Detektionsmittel 4.

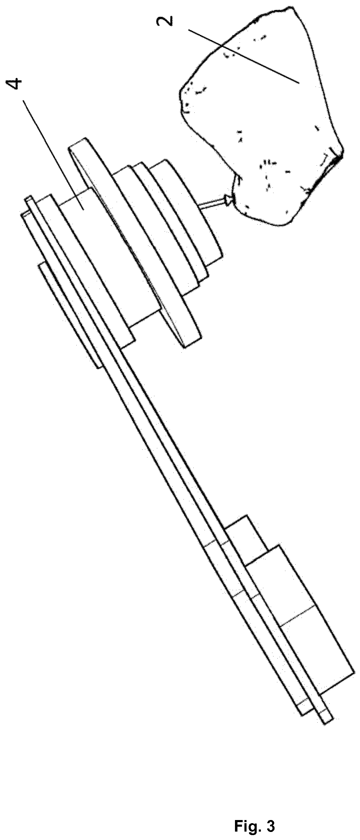

Figur 3 zeigt eine Einzelansicht der Felsenbeinregion des Innenohres 2 als sensibel detektierte Struktur mit einem frei liegenden optoelektronischem Detektionsmittel 4.

In einem Wirbelsäulenmodell als zweitem Ausführungsbeispiel wird mit einer Nadel als operativem Instrument durch eine Nachbildung der Rückenpartie (nicht gezeigt) gestochen. In der Wirbelsäule als austauschbare Übungsregion mit dem in ihr enthaltenen Speicherchip, welcher patientenindividuelle Daten enthält, wird daraufhin geübt, einen Nerv zu treffen.

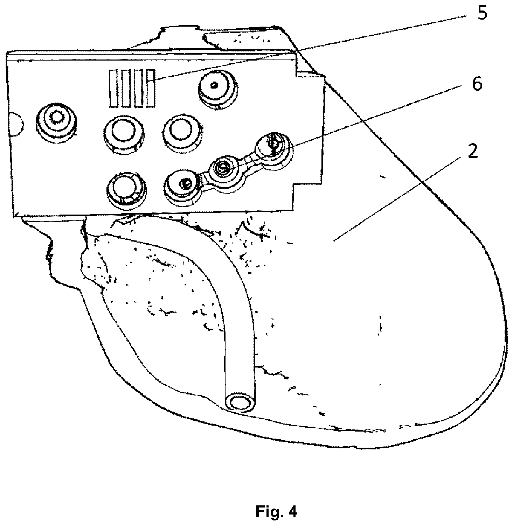

Figur 4 zeigt die austauschbare Übungsregion 2 mit Speichermedium 5 und Anschlüssen 6 für die Mittel zur Signalübertragung an die elektronische Steuer-, Mess- und Auswerteeinheit. Als austauschbare Übungsregion 2 ist die Felsenbeinregion gezeigt. Ein Speichermedium5, hier eine SSD-Karte, ist lösbar in der Rückseite der austauschbaren Übungsregion 2 angeordnet. Weiterhin sind Anschlüsse 6 für die Mittel zur Signalübertragung an die elektronische Steuer-, Mess- und Auswerteeinheit (nicht gezeigt) an der Rückseite der austauschbaren Übungsregion 2 angebracht.

In der Aussparung des Schädels (nicht gezeigt) ist eine (Video-)Digitalkamera (nicht gezeigt) so angeordnet, dass der operative Eingriff durch einen Computer erfasst wird und weiterhin die Positionierung der operativen Instrumente und/oder der Implantate durch den Computer an der Rückseite der Felsenbeinregion überwacht wird. Alle stattfindenden Prozesse werden auf einem Monitor (nicht dargestellt) dargestellt. Die (Video-)Digitalkamera (nicht dargestellt) sendet dabei ein rückgekoppeltes Signal an den Computer (nicht dargestellt).

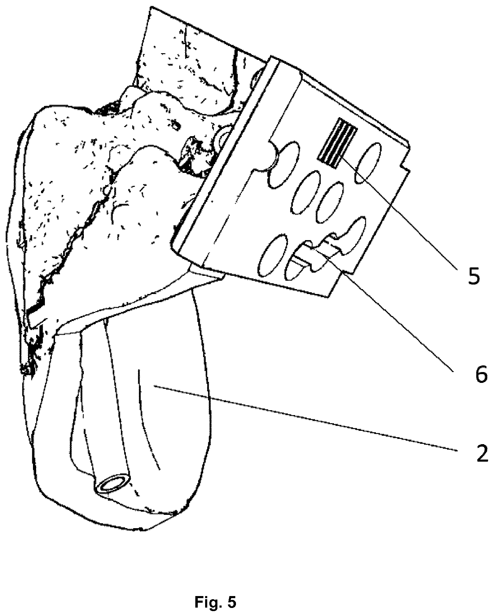

Figur 5 zeigt die austauschbare Felsenbeinregion 2 aus Fig. 4 von der Seite. Auch hier ist wieder die SSD-Karte als Speichermedium 5 zu erkennen, welche lösbar in der Rückseite der austauschbaren Felsenbeinregion 2 angeordnet ist. Weiterhin sind die Anschlüsse 6 für die Mittel zur Signalübertragung an die elektronische Steuer-, Mess- und Auswerteeinheit (nicht gezeigt) an der Rückseite der austauschbaren Felsenbeinregion 2 zu sehen.

In einem Nasenmodell der Nasenregion als zweitem Ausführungsbeispiel werden bspw. mit verschiedenen Instrumenten, Endoskopen und medizinischen Geräten die Siebbeinzellen eröffnet. Der Speicherchip der austauschbaren Übungsregion kann hier bspw. die Patienteninformationen wie Alter, Anamnese, Patientenanatomie und das Verhalten des künstlichen Patienten bei Verletzung einer Risikostruktur enthalten. Des Weiteren können Vorschläge für den weiteren operationsverlauf von dem Speicherchip ausgelesen werden.

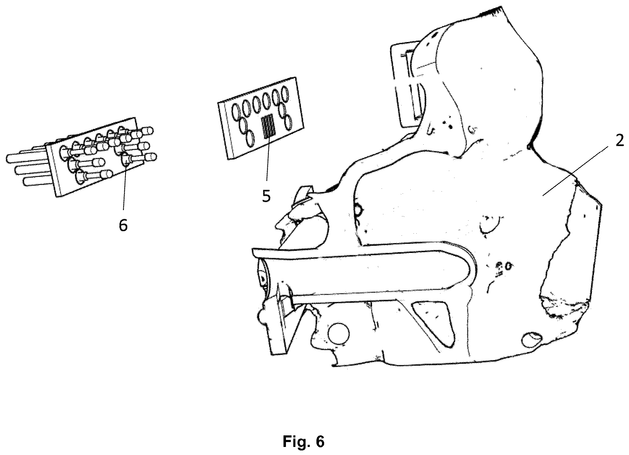

Figur 6 zeigt in einem zweiten Ausführungsbeispiel eine austauschbare Übungsregion 2, welche die Nasenregion darstellt, von der Seite. In der Sprengskizze ebenfalls dargestellt ist das Speichermedium 5, welches als SSD-Karte ausgeführt ist sowie Anschlüsse 6 für die Mittel zur Signalübertragung an die elektronische Steuer-, Mess- und Auswerteeinheit (nicht gezeigt), welche als Pins dargestellt sind.

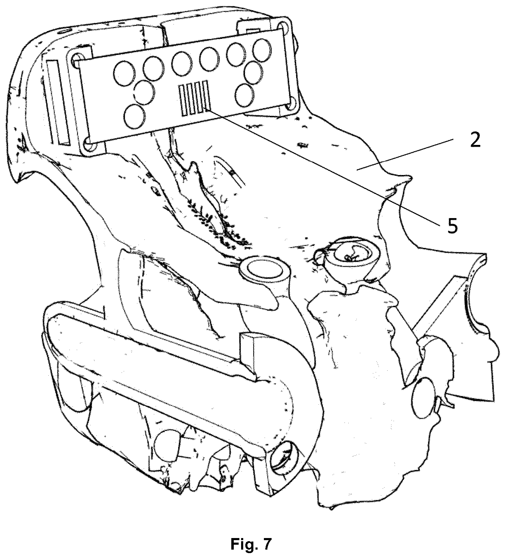

Figur 7 zeigt die austauschbare Nasenregion 2 aus Fig. 6 in einer Frontansicht, nun mit lösbar eingesetzter SSD-Karte als Speichermedium 5.

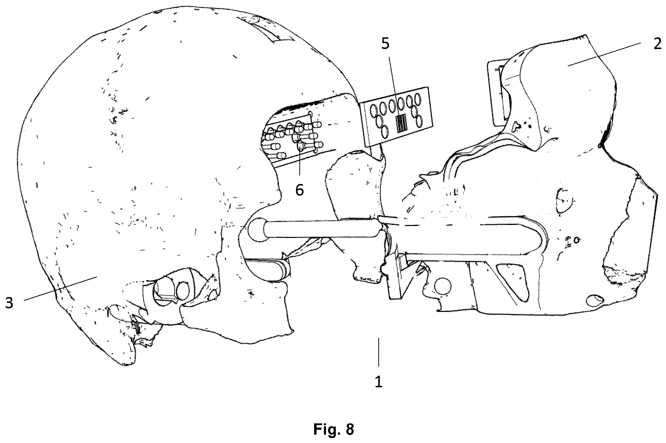

Figur 8 zeigt eine Übersichtsskizze des erfindungsgemäßen Systems 1. Als austauschbare Übungsregion 2 ist die Felsenbeinregion gezeigt. Diese kann als sensibel detektierte Struktur in der anatomischen Nachbildung eines Körperteils 3, hier einem menschlichen Schädel, formschlüssig eingesetzt werden. Eine SSD-Karte als Speichermedium 5 sowie Anschlüsse für die Mittel zur Signalübertragung (nicht gezeigt) an die elektronische Steuer-, Mess- und Auswerteeinheit (nicht gezeigt) sind sprengskizzenartig eingezeichnet und werden lösbar in die austauschbare Nasenregion 2 eingesetzt.

In einem Wirbelsäulenmodell als drittem Ausführungsbeispiel wird mit einer Nadel als operativem Instrument durch eine Nachbildung der Rückenpartie (nicht gezeigt) gestochen. In der Wirbelsäule als austauschbare Übungsregion mit dem in ihr enthaltenen Speicherchip, welcher patientenindividuelle Daten enthält, wird daraufhin geübt, einen Nerv zu treffen.

### Bezugszeichen

- 1: System zur Validierung und zum Training operativer Eingriffe in der Human- und Veterinärmedizin
- 2: austauschbare Übungsregion
- 3: anatomische Nachbildung eines Körperteils
- 4: optoelektronisches Detektionsmittel
- 5: Speichermedium
- 6: Anschlüsse für die Mittel zur Signalübertragung an die elektronische Steuer-, Mess- und Auswerteeinheit

## Patentansprüche

1. System zur Validierung und zum Training operativer Eingriffe in der Human- und Veterinärmedizin (1), welche ein dem menschlichen oder tierischen Körper anatomisch nachgebildetes Trainingsmodell aufweist, umfassend
- eine anatomische Nachbildung eines Körperteils (3) des menschlichen oder tierischen Körpers, welche eine Aussparung aufweist,
- eine anatomische Nachbildung einer austauschbaren Übungsregion (2),
welche in die Aussparung der anatomischen Nachbildung des Körperteils (3) einsetzbar ausgebildet ist, und welche:
- eine Vorderseite aufweist, welche von außen zugänglich ist, und
- eine Rückseite aufweist, welche zumindest teilweise formschlüssig mit der Aussparung der anatomischen Nachbildung des Körperteils (3) verbunden ist, wobei die Rückseite dem Operationsfeld für einen Benutzer des Trainingsmodells, in welchem er operative Eingriffe übt, entspricht und von außen für den Benutzer nicht zugänglich ist, sowie
- ein optoelektronisches Detektionsmittel (4),
**dadurch gekennzeichnet, dass** das optoelektronische Detektionsmittel (4) derart in der Aussparung der anatomischen Nachbildung des Körperteils (3) angeordnet ist, dass dieses zur Erfassung der operativen Eingriffe sowie der Überwachung der Positionierung von operativen Instrumenten und/oder von Implantaten an der Rückseite der austauschbaren Übungsregion (2) ausgebildet ist.

2. System (1) nach Anspruch 1, **dadurch gekennzeichnet, dass** die Erfassung der operativen Eingriffe sowie die Überwachung der Positionierung der operativen Instrumente und/oder der Implantate an der Rückseite der austauschbaren Übungsregion (2) durch eine elektronische Steuer-, Mess- und Auswerteeinheit realisiert wird.

3. System (1) nach Anspruch 1 oder 2, **dadurch gekennzeichnet, dass** es Mittel zur Signalübertragung umfasst.

4. System (1) nach einem der Ansprüche 1 bis 3, **dadurch gekennzeichnet, dass** es sich bei dem optoelektronischen Detektionsmittel (4) um eine Video-Digitalkamera mit einem CMOS-Sensor oder einem CCD-Sensor handelt.

5. System (1) nach einem der Ansprüche 1 bis 4, **dadurch gekennzeichnet, dass** zumindest Bereiche der austauschbaren Übungsregion (2) transparent ausgebildet sind.

6. System (1) nach einem der Ansprüche 1 bis 5, **dadurch gekennzeichnet, dass** die austauschbare Übungsregion (2) ein Speichermedium (5) aufweist, auf welchem patientenindividuelle Daten gespeichert sind und welches mit weiteren patientenindividuellen Daten beschreibbar oder überschreibbar ist, wobei das Speichermedium (5) lösbar in der austauschbaren Übungsregion (2) angeordnet ist.

7. System (1) nach einem der Ansprüche 1 bis 6, **dadurch gekennzeichnet, dass** das optoelektronische Detektionsmittel (4) und/oder das Speichermedium (5) durch Mittel zur Signalübertragung mit der Steuer-, Mess- und Auswerteeinheit verbunden ist.

8. System (1) nach einem der Ansprüche 1 bis 7, **dadurch gekennzeichnet, dass** die austauschbare Übungsregion (2) lösbar mit der anatomischen Nachbildung des Körperteils (3) verbunden ist.

9. Anatomische Nachbildung einer austauschbaren Übungsregion (2), welche in eine Aussparung einer anatomischen Nachbildung eines Körperteils (3) einsetzbar ausgebildet ist,
**dadurch gekennzeichnet, dass** diese in eine Haltevorrichtung einsetzbar ausgebildet ist, und welche:
- eine Vorderseite aufweist, welche von außen zugänglich ist, und
- eine Rückseite aufweist, welche formschlüssig mit der Haltevorrichtung verbunden ist,
wobei die Rückseite dem Operationsfeld für einen Benutzer des Trainingsmodells, in welchem er operative Eingriffe übt, entspricht und von außen für den Benutzer nicht zugänglich ist,
wobei die austauschbare Übungsregion (2) dem Operationsfeld entspricht, ein optoelektronisches Detektionsmittel (4) und ein Speichermedium (5) umfasst und zumindest Bereiche der austauschbaren Übungsregion (2) teilweise transparent ausgebildet sind, wobei das optoelektronische Detektionsmittel (4) derart in der Aussparung der anatomischen Nachbildung des Körperteils (3) angeordnet ist, dass dieses zur Erfassung der operativen Eingriffe sowie der Überwachung der Positionierung von operativen Instrumenten und/oder von Implantaten an der Rückseite der austauschbaren Übungsregion (2) ausgebildet ist,
wobei die Haltevorrichtung als technische Halterung ausgebildet ist.

10. Anatomische Nachbildung einer austauschbaren Übungsregion (2) nach Anspruch 9, **dadurch gekennzeichnet, dass** sie Mittel zur Signalübertragung umfasst.

11. Verfahren zur Validierung und zum Training operativer Eingriffe in der Human- und Veterinärmedizin mit einem System nach einem der Ansprüche 1 bis 8,
aufweisend das den menschlichen oder tierischen Körper anatomisch nachgebildete Trainingsmodell, **dadurch gekennzeichnet, dass** das in der austauschbaren Übungsregion (2) des Trainingsmodells angebrachte optoelektronische Detektionsmittel (4) ein rückgekoppeltes Signal an die Steuer-, Mess- und Auswerteeinheit sendet, wobei das übermittelte Signal Daten über den aktuellen Trainingsverlauf enthält, welche audiovisuelle Spezifikationen wie Bildauflösung und abgeleitetes Seitenverhältnis, Bildwiederholungsrate und Farbtiefe umfassen.

12. Verfahren nach Anspruch 11, **dadurch gekennzeichnet, dass** durch das rückgekoppelte Signal des optoelektronischen Detektionsmittels (4) in Echtzeit der Trainingsverlauf überwacht und beurteilt wird.

13. Verfahren nach einem der Ansprüche 11 oder 12, **dadurch gekennzeichnet, dass** die anatomische Nachbildung des Körperteils (3) mit der austauschbaren Übungsregion (2) verbunden wird und das in der austauschbaren Übungsregion (2) enthaltene Speichermedium (5) von der elektronischen Steuer-, Mess- und Auswerteeinheit durch Mittel zur Signalübertragung erkannt wird.

14. Verwendung eines Systems (1) nach einem der Ansprüche 1 bis 8, einer anatomischen Nachbildung einer austauschbaren Übungsregion (2) nach einem der Ansprüche 9 oder 10 und/oder eines Verfahrens nach einem der Ansprüche 11 bis 13 zur Validierung und/oder zum Training operativer Eingriffe in der Human- und Veterinärmedizin.

## Claims

1. System for the validation and training of surgical interventions in human and veterinary medicine (1), which has a training model anatomically modelled on the human or animal body, comprising
- an anatomical replica of a body part (3) of the human or animal body, which has a recess,
- an anatomical replica of an exchangeable training region (2), which is designed to be insertable into the recess of the anatomical replica of the body part (3), and which:
- has a front side, which is accessible from the outside, and
- has a rear side, which is at least partially positively connected to the recess of the anatomical replica of the body part (3), wherein the rear side corresponds to the surgical field for a user of the training model in which he practices surgical interventions and is not accessible to the user from the outside, and
- an optoelectronic detection means (4),
**characterized in that** the optoelectronic detection means (4) is arranged in the recess of the anatomical replica of the body part (3) in such a way that it is designed to detect the surgical interventions and to monitor the positioning of surgical instruments and/or implants at the rear of the exchangeable training region (2).

2. System (1) according to claim 1, **characterized in that** the recording of the surgical interventions and the monitoring of the positioning of the surgical instruments and/or the implants at the rear of the exchangeable training region (2) is realized by an electronic control, measurement and evaluation unit.

3. System (1) according to claim 1 or 2, **characterized in that** it comprises means for signal transmission.

4. System (1) according to one of claims 1 to 3, **characterized in that** the optoelectronic detection means (4) is a video digital camera with a CMOS sensor or a CCD sensor.

5. System (1) according to one of claims 1 to 4, **characterized in that** at least areas of the exchangeable training region (2) are transparent.

6. System (1) according to one of claims 1 to 5, **characterized in that** the exchangeable training region (2) has a storage medium (5) on which patient-specific data is stored and which is writable or overwritable with further patient-specific data, the storage medium (5) being detachably arranged in the exchangeable training region (2).

7. System (1) according to one of claims 1 to 6, **characterized in that** the optoelectronic detection means (4) and/or the storage medium (5) is connected to the control, measurement and evaluation unit by means for signal transmission.

8. System (1) according to one of claims 1 to 7, **characterized in that** the exchangeable training region (2) is detachably connected to the anatomical replica of the body part (3).

9. Anatomical replica of an exchangeable training region (2), which is designed to be insertable into a recess of an anatomical replica of a body part (3),
**characterized in that** it is designed to be insertable into a holding device, and which:
- has a front side which is accessible from the outside, and
- has a rear side which is positively connected to the holding device,
wherein the rear side corresponds to the surgical field for a user of the training model, in which he practices surgical interventions, and is not accessible to the user from the outside,
wherein the exchangeable training region (2) corresponds to the surgical field, comprises an optoelectronic detection means (4) and a storage medium (5) and at least areas of the exchangeable training region (2) are partially transparent, wherein the optoelectronic detection means (4) is arranged in the recess of the anatomical replica of the body part (3) in such a way that it is designed to detect the surgical interventions and to monitor the positioning of surgical instruments and/or implants on the rear side of the exchangeable training region (2),
wherein the holding device is designed as a technical holder.

10. Anatomical replica of an exchangeable training region (2) according to claim 9, **characterized in that** it comprises means for signal transmission.

11. Method for validation and training of surgical interventions in human and veterinary medicine with a system according to any one of claims 1 to 8,
comprising the training model anatomically simulating the human or animal body, **characterized in that** the optoelectronic detection means (4) mounted in the exchangeable training region (2) of the training model transmits a feedback signal to the control, measurement and evaluation unit, the transmitted signal containing data on the current training progress comprising audiovisual specifications such as image resolution and derived aspect ratio, frame rate and colour depth.

12. Method according to claim 11, **characterized in that** the training progress is monitored and assessed in real time through the feedback signal of the optoelectronic detection means (4).

13. Method according to one of claims 11 or 12, **characterized in that** the anatomical replica of the body part (3) is connected to the exchangeable training region (2) and the storage medium (5) contained in the exchangeable training region (2) is detected by the electronic control, measurement and evaluation unit by means for signal transmission.

14. Use of a system (1) according to any one of claims 1 to 8, of an anatomical replica of an exchangeable training region (2) according to any one of claims 9 or 10 and/or a method according to any one of claims 11 to 13 for validation and/or training of surgical interventions in human and veterinary medicine.

## Revendications

1. Système de validation et d'entraînement d'interventions chirurgicales en médecine humaine et vétérinaire (1), qui présente un modèle d'entraînement reproduisant anatomiquement le corps humain ou animal, comprenant
- une reproduction anatomique d'une partie (3) du corps humain ou animal, qui présente un évidement,
- une réplique anatomique d'une région d'exercice interchangeable (2) qui est formée de manière à pouvoir être insérée dans l'évidement de la réplique anatomique de la partie du corps (3), et qui
- présente une face avant, qui est accessible de l'extérieur, et
- présente une face arrière, qui est reliée au moins partiellement par complémentarité de forme à l'évidement de la reproduction anatomique de la partie du corps (3), la face arrière correspondant au champ opératoire pour un utilisateur du modèle d'entraînement, dans lequel il s'exerce à des interventions chirurgicales, et n'étant pas accessible de l'extérieur pour l'utilisateur, ainsi que
- un moyen de détection optoélectronique (4),
**caractérisé en ce que** le moyen de détection optoélectronique (4) est disposé dans l'évidement de la reproduction anatomique de la partie du corps (3) de telle sorte que celui-ci est conçu pour la détection des interventions chirurgicales ainsi que pour la surveillance du positionnement d'instruments chirurgicaux et/ou d'implants sur la face arrière de la région d'entraînement (2) interchangeable..

2. Système (1) selon la revendication 1, **caractérisé en ce que** la détection des interventions chirurgicales ainsi que la surveillance du positionnement des instruments chirurgicaux et/ou des implants sur la face arrière de la région d'exercice interchangeable (2) sont réalisés par une unité électronique de commande, de mesure et d'évaluation.

3. Système (1) selon la revendication 1 ou 2, **caractérisé en ce qu'**il comprend des moyens de transmission de signaux.

4. Système (1) selon l'une des revendications 1 à 3, **caractérisé en ce que** le moyen de détection optoélectronique (4) est une caméra vidéo numérique comprenant un capteur CMOS ou un capteur CCD.

5. Système (1) selon l'une des revendications 1 à 4, **caractérisé en ce qu'**au moins des zones de la région d'exercice interchangeable (2) sont conçues d'être transparent.

6. Système (1) selon l'une des revendications 1 à 5, **caractérisé en ce que** la région d'exercice (2) interchangeable présente un support de mémoire (5) sur lequel sont enregistrées des données propres au patient et sur lequel on peut écrire ou réécrire d'autres données propres au patient, le support de mémoire (5) étant disposé de manière amovible dans la région d'exercice (2) interchangeable.

7. Système (1) selon l'une des revendications 1 à 6, **caractérisé en ce que** le moyen de détection optoélectronique (4) et/ou le support de mémoire (5) est relié à l'unité de commande, de mesure et d'évaluation par des moyens de transmission de signaux.

8. Système (1) selon l'une des revendications 1 à 7, **caractérisé en ce que** la région d'exercice interchangeable (2) est reliée de manière amovible à la réplique anatomique de la partie du corps (3).

9. Reproduction anatomique d'une région d'exercice interchangeable (2), qui est formée de manière à pouvoir être insérée dans un évidement d'une reproduction anatomique d'une partie du corps (3),
**caractérisée en ce qu'**elle est conçue pour être insérée dans un dispositif de maintien, et qui
- présente une face avant qui est accessible de l'extérieur, et
- présente une face arrière qui est reliée par complémentarité de formes au dispositif de maintien,
la face arrière correspondant au champ opératoire pour un utilisateur du modèle d'entraînement, dans lequel il s'exerce à des interventions chirurgicales, et n'étant pas accessible de l'extérieur pour l'utilisateur,
la région d'exercice interchangeable (2) correspondant au champ opératoire, comprenant un moyen de détection optoélectronique (4) et un support de mémoire (5), et au moins des zones de la région d'exercice interchangeable (2) conçues d'être partiellement transparentes, le moyen de détection optoélectronique (4) étant disposé dans l'évidement de la reproduction anatomique de la partie du corps (3) de telle sorte que celui-ci est conçu pour la détection des interventions chirurgicales ainsi que pour la surveillance du positionnement d'instruments chirurgicaux et/ou d'implants sur la face arrière de la région d'exercice interchangeable (2),
le dispositif de maintien étant conçu comme un support technique.

10. Reproduction anatomique d'une région d'exercice interchangeable (2) selon la revendication 9, **caractérisée en ce qu'**elle comprend des moyens de transmission de signaux.

11. Procédé de validation et d'entraînement d'interventions chirurgicales en médecine humaine et vétérinaire avec un système selon l'une des revendications 1 à 8, présentant le modèle d'entraînement reproduisant anatomiquement le corps humain ou animal, **caractérisé en ce que** le moyen de détection optoélectronique (4) placé dans la région d'entraînement interchangeable (2) du modèle d'entraînement envoie un signal de rétroaction à l'unité de commande, de mesure et d'évaluation, le signal transmis contenant des données sur le déroulement actuel de l'entraînement, qui comprennent des spécifications audiovisuelles telles que la résolution d'image et le rapport d'aspect dérivé, le taux de répétition d'image et la profondeur de couleur.

12. Procédé selon la revendication 11, **caractérisé en ce que** le déroulement de l'entraînement est surveillé et évalué en temps réel par le signal de rétroaction du moyen de détection optoélectronique (4).

13. Procédé selon l'une des revendications 11 ou 12, **caractérisé en ce que** la reproduction anatomique de la partie du corps (3) est reliée à la région d'exercice interchangeable (2) et le support de mémoire (5) contenu dans la région d'exercice interchangeable (2) est reconnu par l'unité électronique de commande, de mesure et d'évaluation par des moyens de transmission de signaux.

14. Utilisation d'un système (1) selon l'une des revendications 1 à 8, d'une reproduction anatomique d'une région d'exercice interchangeable (2) selon l'une des revendications 9 ou 10 et/ou d'un procédé selon l'une des revendications 11 à 13 pour la validation et/ou l'entraînement d'interventions chirurgicales en médecine humaine et vétérinaire.
